# EUROPEAN PATENT SPECIFICATION

(11) **EP 0 738 502 B1**
(45) Date of publication and mention of the grant of the patent: **04.09.2002**
(21) Application number: 96302737.0
(22) Date of filing: 19.04.1996
(51) Int. Cl.: A61B 17/72

(54) **Bone support to be inserted in the interior of a bone**
Intramedulläre Knochenstütze
Support intramédullaire d'un os

(30) Priority: 20.04.1995 GB 9508033
(43) Date of publication of application: 23.10.1996
(73) Proprietor: HALIFAX ORTHOPAEDIC RESEARCH LIMITED, Halifax, West Yorkshire HX1 2BX (GB)
(72) Inventor: Halder, Subhash Chandra, Halifax, West Yorkshire HX1 2BX (GB)
(74) Representative: Neill, Alastair William

(56) References cited:
- EP-A- 0 561 295
- DE-A- 2 404 441
- US-A- 2 998 007

## Description

The present invention relates to a bone support.

Fractured or otherwise damaged bones have traditionally been supported externally, for example by encasing the whole or part of a limb in the plaster cast, whilst the bone inside the limb heals. In recent years, apparatus for supporting the bone from within the bone itself has become known. One such apparatus is the subject of our co-pending United Kingdom patent application number 92 11445.3 now granted as GB 2 256 802.

EP-A-0 561 295 discloses bone support apparatus comprising an elongate bone support member having a proximal end, a distal end, a substantially hollow interior, and an outer periphery, the support member being arranged, in use, to be inserted into the interior of a bone, the apparatus further comprising a fixing bracket attached to the support member, the bracket defining at least one aperture arranged, in use, to receive a fixing member for fixing the support member relative to the bone.

However, EP-A-0 561 295 has the disadvantage that the bracket is separate from the bone support member. The fixing bracket is connected transversely to the distal end of the support member via a screw and thus may have slight movement with respect to the support member.

A further disadvantage is that the fixing bracket sits on the end of the bone fixed in place by screws. As a result any load caused by the rotation of the support member relative to the bone is borne fully by the fixing screws. Furthermore, there could be relative movement between the support member and the fixing screws unless the screws are a tight fit in the apertures in the fixing bracket.

The present invention is characterised in that the fixing bracket is rigidly attached to the outer periphery of the support member adjacent to the distal end, projecting beyond, and substantially tangentially to, the support member in two substantially opposed directions to form wing projections, that, in use, the bracket extends over the outer periphery of the bone thus resisting rotational movement of the support member within the bone.

The present invention has the advantage that, in use, the fixing bracket may sit against the bone such that loads caused by rotation of the support member within the bone may be borne by the fixing bracket pressing on the bone. As a result rotation of the support member relative to the bone may be without said loads being borne solely by a fixing member.

The fixing bracket may be welded to the support member.

Preferably, the substantially hollow elongate bone support member may be arranged, in use, to receive an elongate projection, the elongate projection being arranged, in use, to be inserted into the distal end of the bone support member and to extend along the bone support member, the bone support member further comprising a plurality of apertures towards the proximal end thereof, the projection comprising a plurality of portions arranged respectively to extend through the apertures in the bone support member to engage the bone.

Preferably, the bone support member may comprise three apertures and the elongate projection may comprise three portions.

The bone support member may comprise deflector means arranged, in use, to deflect each portion of the projection so that each portion of the projection is guided through the respective apertures in the bone support member during insertion of the projection.

The proximal end of the hollow bone support member may be substantially closed by a closure member within the bone support member.

Preferably the proximal end of the hollow bone support member may be substantially closed by a first end of a closure member within the bone support member and a second end of the closure member substantially opposed to the first end, may comprise the deflector means.

The proximal end of the bone support member may be substantially blunt, such that it displaces cancellous bone during insertion of the bone support member along the bone.

The fixing member may comprise a screw arranged to pass through the aperture in the fixing bracket to engage a portion of the bone.

The fixing bracket may comprise two apertures and two fixing members, which may comprise screws, may be arranged to pass through the apertures in the fixing bracket to engage the bone.

The invention may be carried into practice in various ways, but an embodiment will now be described by way of example only, with reference to the accompanying diagrammatic drawings in which:
Figure 1 shows in front, part-sectional view bone support apparatus according to an embodiment of the present invention;
Figure 2 shows a projection means for use with the apparatus of Figure 1;
Figure 3 shows the bone support apparatus of Figure 1 in an alternative configuration; and
Figure 4 shows the bone support apparatus of Figure 1 in side view.

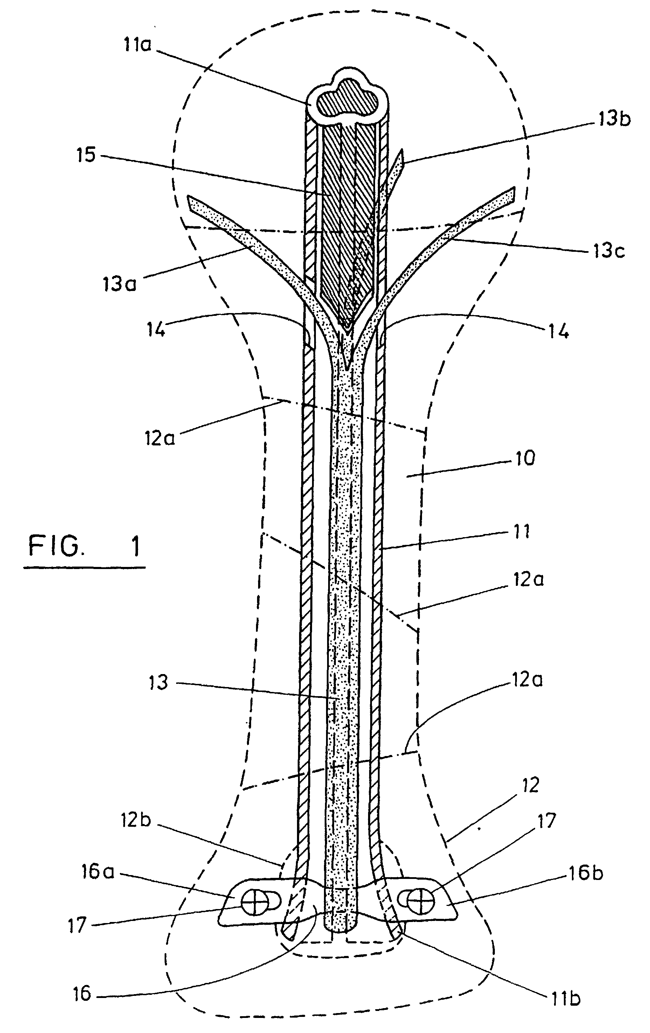

Referring particularly to Figure 1, there is shown generally at 10 bone support apparatus comprising an elongate steel bone support 11, which is substantially hollow and of generally clover-leaf cross-section in shape. The bone support 11 is shown located within a humerus bone 12 shown in broken lines. Fractures in the bone 12 are schematically represented at 12a. An aperture 12b is shown in the bone 12 at a distal end. After the aperture 12b is made, the bone support 11 is inserted into the bone 12.

Within the hollow bone support 11 is located a projection member 13 having three portions 13a, 13b and 13c. Portion 13a projects to the left of the bone support 11 as shown, portion 13b projects into the drawing and portion 13c projects to the right of the bone support member 11 as shown.

The bone support 11 is provided with three apertures 14 through which the portions 13a, 13b and 13c project in use, to engage the interior of the bone 12.

Within the bone support 11, slightly beyond the apertures 14, is located a deflector member 15, which comprises a solid steel rod, of generally clover-leaf cross-section, which substantially fills the cavity of the bone support 11 and thereby substantially closes a proximal end 11a of the bone support. The deflector 15 is shaped to form three deflecting faces in the region of the apertures 14 and so guides the portions 13a, 13b and 13c through their respective apertures as the projection 13 is inserted from a distal end 11b of the bone support.

Close to the distal end 11b of the bone support 11 is a steel fixing bracket 16 which is welded onto the support 11. The bracket projects beyond the support 11 in opposed directions to form two wing projections 16a and 16b which in use are located outside of the aperture 12b of the bone 12, and against the exterior of the bone. Each of the wing projection 16a and 16b is provided with an aperture (not shown) through which screws 17 are arranged, in use, to pass.

The screws pass through the wings 16a and 16b and into the cortex of the bone 12. They then pass through the interior of the bone and into the cortex on the other side of the bone 12.

By means of the screws 17 the bone support 11 is fixed relative to the bone 12 at the distal end 11b. The portions 13a, 13b and 13c, which project outwards from apertures 14 in the bone support 11 engage the cancellous bone (marrow) in the region of the proximal end 11a of the bone support, thus fixing the bone support 11 relative to the bone 12 at the proximal end 11a.

Since both proximal and distal ends of the bone support 11 are fixed relative to the bone 12, the bone 12 is prevented from rotation about one or more of the fractures represented schematically 12a, and is thus able to heal in the correct orientation.

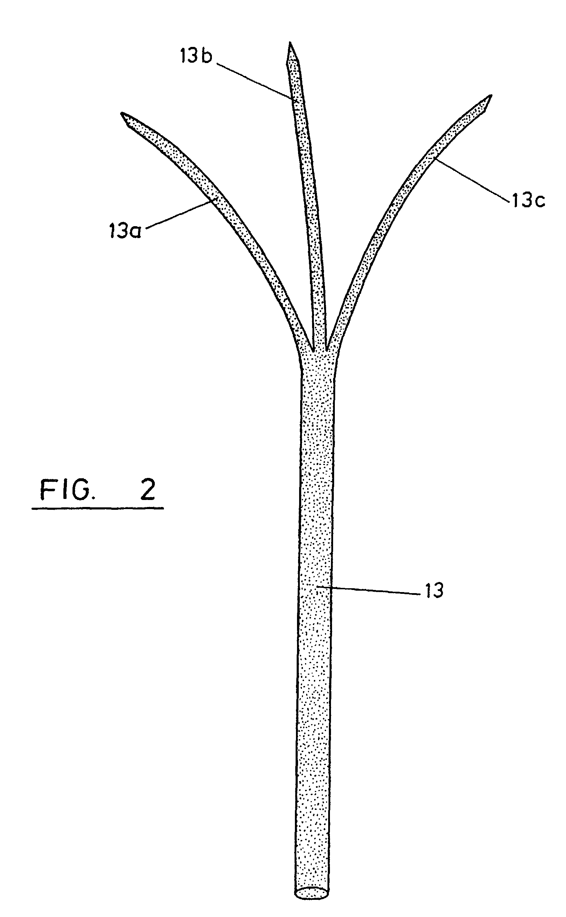

Turning to Figure 2, this shows the projection 13 with portions 13a, 13b and 13c. The projection 13 may be manufactured from steel and may comprise three separate wires welded or brazed together, or may comprise a single wire having three portions 13a, 13b and 13c split and bent slightly apart. The ends of the portions 13a, 13b and 13c are pointed to enable them to pierce the cancellous bone.

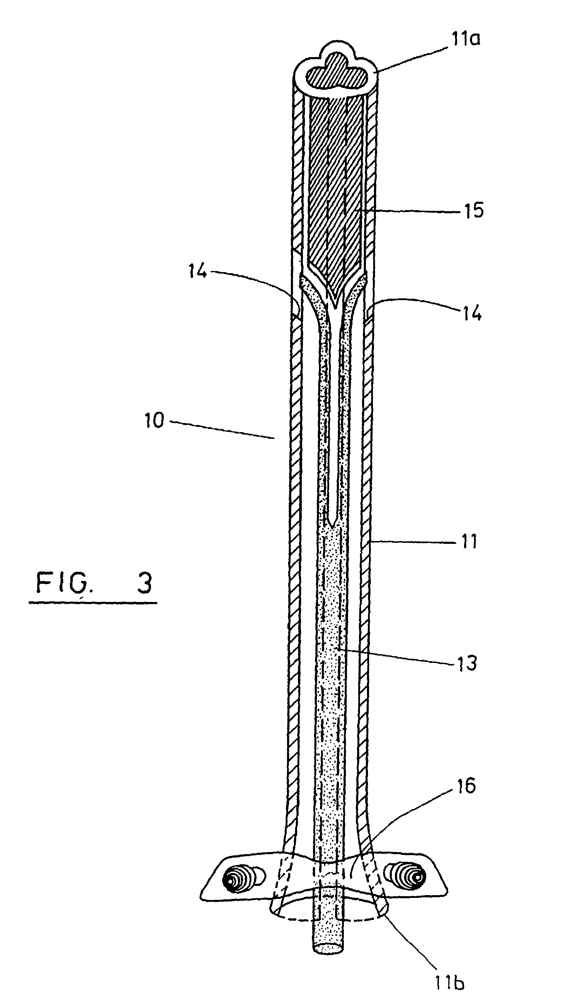

Figure 3 shows the bone support 11 during insertion of the projection 13. Only portions 13a and 13c are shown, since portion 13b is located towards the rear of the bone support 11.

Initially, the projection 13 is inserted in the bone support 11 at the distal end 11b. When the portions 13a, 13b and 13c reach the deflector member 15, they are each deflected through a separate aperture 14 in the bone support 11. The edges of the apertures 14 are bevelled to facilitate exit of the portions 13a, 13b and 13c through the apertures 14. As the projection 13 is pushed further into the bone support 11, the portions 13a, 13b and 13c are forced outwards, away from the bone support 11 and into the cancellous bone (not shown) to become fixed there in three separate locations.

The closed proximal end 11a of the bone support 11 is polished and substantially blunt so that during insertion of the bone support 11 in the bone 12 the cancellous bone is displaced, but there is no risk of the cortex (harder, outer bone) being pierced by a bone support which is inserted too far into the bone.

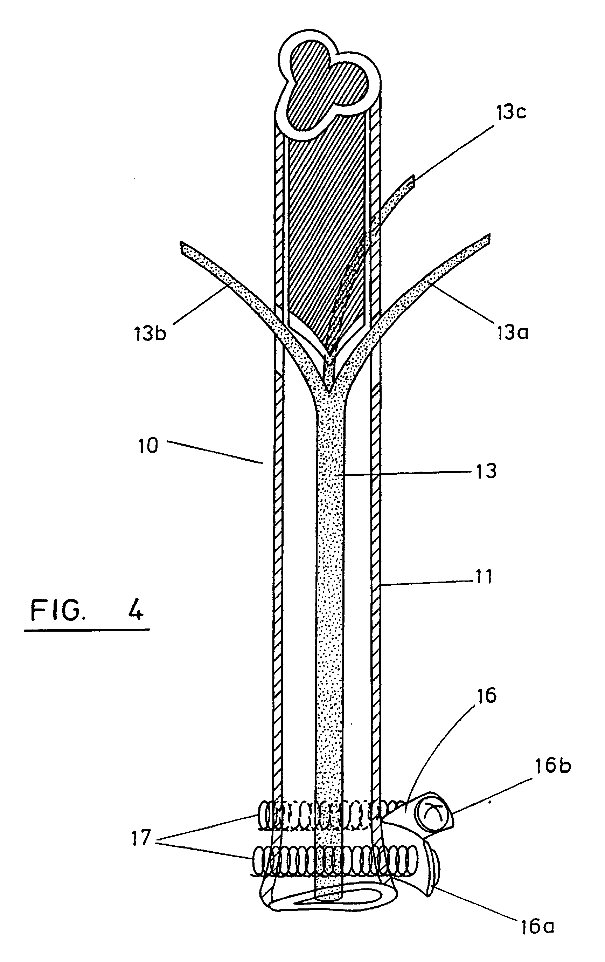

Figure 4 is a side view of the bone support apparatus 10 showing the two screws 17 passing through the wings 16a and 16b of the bracket 16.

In use, the bone support 11 is inserted in the bone 12 through aperture 12b to the required depth and the projection 13 is then inserted into the bone support 11. When inserting the bone support 11, it may be advantageous to engage the distal end 11b with an elongate threaded member (not shown). A portion of the distal end 11b of the bone support 11 may be cylindrical and may be internally threaded for this purpose. This enables the bone support to be pushed deeper into the bone 12.

The portions 13a, 13b and 13 are deflected out of apertures 14 by the deflector means 15 and engage the cancellous bone, in the region of the proximal end 11a of the bone support 11.

Screws 17 are then passed through the wings 16a and 16b of the bracket 16, into the cortex on one side of the bone, through the interior of the bone and are screwed into the cortex on the other side of the bone 12. The fractured bone is then unable to rotate during healing. Once the bone 12 is healed, the projection 13 is withdrawn using forceps, the screws 17 are removed, and then the bone support 11 is withdrawn from the bone. This may conveniently be effected by engaging the apertures (not shown) in the bracket 16 with appropriate withdrawal means (not shown).

The invention provides a secure bone support tor a fractured or otherwise damaged bone in which the correct location of the projection 13 is effected by use of the deflector 15. The bracket 16 enables the use of screws to fix the distal end 11b without the necessity to pass through the interior of the bone support 11 itself, thus avoiding interference with the projection 13, and allowing the projection 13 to occupy the interior of the bone support member 11 in the region of the distal end 11b, and it is also useful for withdrawing the bone support once the bone has healed.

It will be understood by the skilled person that the invention may be modified without departing from its scope. For example, the number of projecting portions may be other than three, although this has been found in practice to be a particularly advantageous number of projecting portions. Also it will be understood that, although the invention has been described above in relation to humerus bone, it could equally be employed in other bones of a similar type.

In an alternative embodiment, the proximal end 11a of the bone support 11 may be tapered to facilitate movement of the bone support through the bone, but the taper should have a blunt end rather than a sharp end, so that there is no risk of piercing the cortex.

## Claims

1. Bone support apparatus (10) comprising an elongate bone support member (11) having a proximal end (11a), a distal end (11b), a substantially hollow interior, and an outer periphery, the support member (11) being arranged, in use, to be inserted into the interior of a bone (12), the apparatus further comprising a fixing bracket (16), the bracket (16) defining at least one aperture arranged in use, to receive a fixing member (17) for fixing the support member (11) relative to the bone (12), **characterised in that** the fixing bracket (16) is rigidly attached to the outer periphery of the support member (11) adjacent to the distal end (11b), projecting beyond, and substantially tangentially to, the support member (11) in two substantially opposed directions to form wing projections (16a, 16b), such that, in use, the bracket (16) extends over the outer periphery of the bone (12) thus resisting rotational movement of the support member (11) within the bone (12).

2. Bone support apparatus (10) as claimed in Claim 1, in which the fixing bracket (16) is welded to the support member (11).

3. Bone support apparatus (10) as claimed in Claim 1 or 2, wherein the substantially hollow elongate bone support member (11) is arranged, in use, to receive an elongate projection (13), the elongate projection (13) being arranged, in use, to be inserted into the distal end (11b) of the bone support member (11) and to extend along the bone support member (11), the bone support member (11) further comprising a plurality of apertures (14) towards the proximal end (11a) thereof, the projection (13) comprising a plurality of portions (13a, 13b, 13c) arranged respectively to extend through the apertures (14) in the bone support member (11) to engage the bone (12).

4. Bone support apparatus (10) as claimed in Claim 3, wherein the bone support member (11) comprises three apertures (14) and the elongate projection (13) comprises three portions (13a, 13b, 13c).

5. Bone support apparatus (10) as claimed in Claim 3 or 4 wherein the bone support member (11) comprises deflector means (15) arranged, in use, to deflect each portion (13a, 13b, 13c) of the projection so that each portion (13a, 13b, 13c) of the projection (13) is guided through the respective apertures (14) in the bone support member (11) during insertion of the projection (13).

6. Bone support apparatus (10) as claimed in any preceding claim, wherein the proximal end (11a) of the hollow bone support member (11) is substantially closed by a closure member within the bone support member (11).

7. Bone support apparatus (10) as claimed in Claim 5, wherein the proximal end (11a) of the hollow bone support member (11) is substantially closed by a first end of a closure member within the bone support member (11) and a second end of the closure member substantially opposed to the first end, comprises the deflector means (15).

8. Bone support apparatus (10) as claimed in any preceding claim, wherein the proximal end (11a) of the bone support member (11) is substantially blunt, such that it displaces cancellous bone during insertion of the bone support member (11) along the bone (12).

9. Bone support apparatus (10) as claimed in any preceding claim in which the fixing member (17) comprises a screw arranged to pass through the aperture in the fixing bracket (16) to engage a portion of the bone (12).

## Patentansprüche

1. Knochenstützvorrichtung (10), welche ein längliches Knochenstützglied (11) mit einem proximalen Ende (11a), einem distalen Ende (11b), einem im Wesentlichen hohlen Inneren und einem äußeren Umfang aufweist, wobei das Knochenstützglied (11) so angeordnet ist, dass es beim Gebrauch in das Innere eines Knochens (12) eingesetzt werden kann und wobei die Vorrichtung weiterhin eine Befestigungsschelle (16) aufweist, wobei die Schelle (16) wenigstens eine Öffnung bildet, die im Gebrauch so angeordnet ist, dass sie ein Befestigungsglied (17) zum Befestigen des Stützgliedes (11) relativ zu dem Knochen aufzunehmen vermag, **dadurch gekennzeichnet, dass** die Befestigungsschelle (16) fest mit dem äußeren Umfang des Stützgliedes (11) angrenzend an dem distalen Ende (11b) verbunden ist und das Stützglied (11) im Wesentlichen tangential und in zwei im Wesentlichen entgegengesetzten Richtungen überragt, um flügelartige Verlängerungen (16a, 16b) zu bilden, so dass die Schelle (16) sich im Gebrauch über den äußeren Umfang des Knochens (12) erstreckt, um auf diese Weise sich einer Drehbewegung des Stützgliedes (11) innerhalb des Knochens (12) zu widersetzen.

2. Knochenstützvorrichtung (10) nach Anspruch 1, bei der die Befestigungsschelle (16) mit dem Stützglied (11) verschweißt ist.

3. Knochenstützvorrichtung (10) nach Anspruch 1 oder 2, wobei das im Wesentlichen hohle, längliche Knochenstützglied (11) so angeordnet ist, um im Gebrauch eine längliche Verlängerung (13) aufzunehmen, wobei im Gebrauch die längliche Verlängerung (13) so angeordnet ist, dass sie in das distale Ende (11b) des Knochenstützgliedes (11) eingeführt werden kann und sich entlang des Knochenstützgliedes (11) erstreckt, wobei das Knochenstützglied (11) außerdem eine Vielzahl von zum proximalen Ende (11a) gerichteten Öffnungen (14) aufweist und wobei die Verlängerung (13) eine Vielzahl von Teilabschnitten (13a, 13b, 13c) aufweist, die jeweils so angeordnet sind, dass sie durch die Öffnungen (14) des Knochenstützgliedes (11) hindurchragen, um in den Knochen (12) einzudringen.

4. Knochenstützvorrichtung (10) nach Anspruch 3, wobei das Knochenstützglied (11) drei Öffnungen (14) aufweist und die längliche Verlängerung (13) drei Teilabschnitte (13a, 13b, 13c) aufweist.

5. Knochenstützvorrichtung (10) nach Anspruch 3 oder 4, wobei das Knochenstützglied (11) Ablenkmittel (15) aufweist, das derart angeordnet ist, dass es im Gebrauch jeden Teilabschnitt (13a, 13b, 13c) der Verlängerung so ablenkt, dass beim Einführen der Verlängerung (13) jeder Teilabschnitt (13a, 13b, 13c) der Verlängerung (13) durch die jeweilige Öffnung (14) im Knochenstützglied (11) geführt wird.

6. Knochenstützvorrichtung (10) nach einem der vorhergehenden Ansprüche, wobei das proximale Ende (11a) des hohlen Knochenstützgliedes (11) im Wesentlichen durch ein Schließglied innerhalb des Knochenstützgliedes (11) verschlossen ist.

7. Knochenstützvorrichtung (10) nach Anspruch 5, wobei das proximale Ende (11a) des hohlen Knochenstützgliedes (11) im Wesentlichen durch ein erstes Ende eines Schließgliedes innerhalb des Knochenstützgliedes (11) verschlossen wird, und wobei ein zweites Ende des Schließgliedes, das dem ersten Ende im Wesentlichen gegenüberliegt, das Ablenkmittel (15) aufweist.

8. Knochenstützvorrichtung (10) nach einem der vorhergehenden Ansprüche, wobei das proximale Ende (11a) des Knochenstützgliedes (11) im Wesentlichen stumpf ist, so dass es beim Einführen des Knochenstützgliedes (11) entlang des Knochens (12) den spongiösen Knochen versetzt.

9. Knochenstützvorrichtung (10) nach einem der vorhergehenden Ansprüche, wobei das Befestigungsmittel (17) eine Schraube aufweist, die so angeordnet ist, dass sie durch die Öffnung in der Befestigungsschelle (16) hindurchführt, um in einen Abschnitt des Knochens (12) einzugreifen.

## Revendications

1. Appareil de support (10) pour os comprenant un élément de support allongé (11) pour os ayant une extrémité proximale (11a), une extrémité distale (11b), un intérieur sensiblement creux, et une périphérie extérieure, l'élément de support (11) étant disposé, en service, de manière à être inséré à l'intérieur d'un os (12), l'appareil comprenant en outre une attache de fixation (16), l'attache (16) définissant au moins un orifice disposé, en service, de manière à recevoir un élément de fixation (17) pour fixer l'élément de support (11) par rapport à l'os (12), **caractérisé en ce que** l'attache de fixation (16) est rigidement immobilisée sur la périphérie extérieure de l'élément de support (11) en une position adjacente à l'extrémité distale (11b), et en se projetant au-delà de l'élément de support (11) et sensiblement tangentiellement à celui-ci, dans deux directions sensiblement opposées de manière à former des projections en forme d'ailes (16a, 16b) de sorte que, en service, l'attache (16) s'étende sur la périphérie extérieure de l'os (12), résistant ainsi au mouvement de rotation de l'élément de support (11) au sein de l'os (12).

2. Appareil de support (10) pour os selon la revendication 1, dans lequel l'attache de fixation (16) est soudée à l'élément de support (11).

3. Appareil de support (10) pour os selon la revendication 1 ou 2, dans lequel l'élément de support allongé sensiblement creux (11) pour os est disposé, en service, de manière à recevoir une projection allongée (13), la projection allongée (13) étant disposée, en service, de manière à être insérée dans l'extrémité distale (11b) de l'élément de support (11) pour os et à s'étendre le long de l'élément de support (11) pour os, l'élément de support (11) pour os comprenant en outre une pluralité d'orifices (14) vers l'extrémité proximale (11a) de celui-ci, la projection (13) comprenant une pluralité de portions (13a, 13b, 13c) disposées respectivement de manière à s'étendre au travers des orifices (14) dans l'élément de support (11) pour os pour venir en prise avec l'os (12).

4. Appareil de support (10) pour os selon la revendication 3, dans lequel l'élément de support (11) pour os comprend trois orifices (14) et la projection allongée (13) comprend trois portions (13a, 13b, 13c).

5. Appareil de support (10) pour os selon la revendication 3 ou 4, dans lequel l'élément de support (11) pour os comprend un moyen de déviation (15) disposé, en service, de manière à dévier chaque portion (13a, 13b, 13c) de la projection de sorte que chaque portion (13a, 13b, 13c) de la projection (13) soit guidée au travers des orifices respectifs (14) dans l'élément de support (11) pour os au cours de l'insertion de la projection (13).

6. Appareil de support (10) pour os selon l'une quelconque des revendications précédentes, dans lequel l'extrémité proximale (11a) de l'élément de support creux (11) pour os est sensiblement fermée par un élément de fermeture au sein de l'élément de support (11) pour os.

7. Appareil de support (10) pour os selon la revendication 5, dans lequel l'extrémité proximale (11a) de l'élément de support creux (11) pour os est sensiblement fermée par une première extrémité d'un élément de fermeture au sein de l'élément de support (11) pour os, et une deuxième extrémité de l'élément de fermeture, sensiblement opposée à la première extrémité, comprend le moyen de déviation (15).

8. Appareil de support (10) pour os selon l'une quelconque des revendications précédentes, dans lequel l'extrémité proximale (11a) de l'élément de support (11) pour os est sensiblement émoussée, de sorte qu'elle déplace l'os à éliminer au cours de l'insertion de l'élément de support (11) pour os le long de l'os (12).

9. Appareil de support (10) pour os selon l'une quelconque des revendications précédentes dans lequel l'élément de fixation (17) comprend une vis disposée de manière à passer au travers de l'orifice dans l'attache de fixation (16) et à venir en prise avec une portion de l'os (12).
